(19) Europäisches Patentamt
European Patent Office
Office européen des brevets

(11) EP 1 082 092 B1

(12) **EUROPEAN PATENT SPECIFICATION**

(45) Date of publication and mention of the grant of the patent:
**02.11.2005 Bulletin 2005/44**

(21) Application number: **99924528.5**

(22) Date of filing: **27.05.1999**

(51) Int Cl.7: **A61K 7/40**, A61K 7/50, A61K 7/48, A61K 31/745

(86) International application number:
**PCT/US1999/011730**

(87) International publication number:
**WO 1999/062474 (09.12.1999 Gazette 1999/49)**

# (54) ANTI-GERM ATTACHMENT - COMPOSITION

ZUSAMMENSETZUNG ZUR VERHINDERUNG DER KEIMBINDUNG

COMPOSITION ANTIFIXATION DE GERMES

(84) Designated Contracting States:
**AT BE CH DE DK ES FI FR GB GR IE IT LI NL PT SE**
Designated Extension States:
**RO**

(30) Priority: **01.06.1998 US 87533 P**
**07.01.1999 US 226304**
**04.05.1999 US 304098**

(43) Date of publication of application:
**14.03.2001 Bulletin 2001/11**

(73) Proprietor: **Colgate-Palmolive Company**
**New York, N.Y. 10022 (US)**

(72) Inventors:
- **ANSARI, Shamim, Alam**
**Princeton, NJ 08540 (US)**
- **POLEFKA, Thomas, Gregory**
**Somerset, NJ 08873 (US)**

(74) Representative: **Prins, Adrianus Willem et al**
**Vereenigde,**
**P.O.Box 87930**
**2508 DH Den Haag (NL)**

(56) References cited:
**EP-A- 0 395 215**
**WO-A-96/20993**
**WO-A-97/16168**
**WO-A-98/05294**
**US-A- 4 849 211**
**US-A- 5 110 593**
**US-A- 5 204 093**
**US-A- 5 869 071**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 082 092 B1

Printed by Jouve, 75001 PARIS (FR)

## Description

Basic skin cleansing compositions have been long addressed by the personal care industry. The consumer population is looking for additional benefit beyond basic skin cleansing which now includes germ as well as soil removal. Recently, intensified focus has been directed to the spread of germs from touching various objects in public use such as an ATM machine, public phones, public restrooms, the gym and the like.

EP-A-0 395 215 describes protective gel compositions for coating skin surfaces and in particular a sterilizable water-insoluble gel comprising a major portion of a cosmetically acceptable wax or a synthetic lanolin substitute; a liquid silicone as water-repellent agent; a polypropylene glycol fatty acid ester; and a particulate of polytetrafluorethylene powder.

In WO-97/16168, a personal cleansing composition is described comprising one or more surfactants; a hydrophobic active component which may be a silicone; a hydrocarbon containing component and a cationic polymer. In order to achieve an antibacterial effect, this reference teaches to include antibacterial agents.

New compositions which fight germs have recently been marketed. However, many of these products use high quantities of alcohol to accomplish the degerming of the skin. These products are directed to eliminating preexisting germs present in the skin prior to treatment. Therefore, a need exists for obtaining a longer lasting "antigerm" effect with a cleansing composition normally employed as a rinse-off product which inhibits the further attachment of germs to the skin following the rinse off process.

Such a product should not be restricted to having activity against only one or a small number of germs which can be present on the skin. It should be readily applicable to a large number of germs, regardless of their gram negative or gram positive nature or whatever type of classification system under which they may be categorized. A few compositions with active agents seem to work by stopping the attachment of specific germs to the skin. However, compositions containing these agents are disclosed to be germ specific to only one or a small number of germs.

It has now been discovered that a relatively simple rinse off skin cleansing composition has the ability to inhibit the attachment of germs to the skin for a significant period of time after rinsing the skin. A broad spectrum of germs can be inhibited. In this manner a standard rinse off skin cleansing composition provides a desired benefit to the everyday skin washing population. Additionally, it can provide a meaningful benefit to those individuals whose skin is in particular need to have diminished levels of germs thereon, for example those people suffering from atopic dermatitis, psoriasis, immunodeficient conditions and the like.

A further advantage of the composition is that germs which are present, can be more readily removed from the skin by rinsing with water after treatment of the skin with the composition of the invention. This occurs for a period of time after the initial rinse-off of the composition has occurred.

## SUMMARY OF THE INVENTION

In accordance with the invention, a composition is used for the preparation of a skin cleansing material which inhibits the attachment of germs to the skin, said composition comprising:

(a) a skin cleansing effective amount of 3 to 30 wt.% when used in a liquid composition, or of 60 to 90 wt.% when used in a solid composition of a surfactant or mixture of surfactants,
(b) a hydrocarbonaceous component selected from wax, petrolatum, mineral oil, beeswax, permethyl, lanolin, lanoleic material, coco butter, shea butter and long chain alkyl ester and ether of lanolin in amounts of 0.1 to 8 wt.% of the composition effective to inhibit attachment of germs to the skin,
(c) a cationic polymer; and
(d) a silicone.

The composition can work its effects in many realistic situations. It can reduce the spread of germs from inanimate objects, for example door knobs, phones, water faucets and the like as well as through skin to skin contact, for example the shaking of hands. In summary, the transmission of germs to skin can be reduced by prior contact of skin with the composition of this invention.

## DETAILED DESCRIPTION OF THE INVENTION

The term "germ" as used in the specification and claims of this invention means bacteria and viruses, particularly bacteria. Examples of bacteria which are inhibited from attaching to the skin include Staphylococcus aureus, Staphylococcus epidermis, Corynebacterium minutissium, Escherichia coli, Salmonella, Choleraesu is and Serratia marcescens. Examples of viruses include human rhinovirus and human rotovirus.

The surfactants which can be used in the composition include the following families: anionic, amphoteric,

nonionic and cationic, alone or in combination. Soap, a long chain alkyl or alkenyl, branched or normal carboxylic acid salt such as sodium, potassium, ammonium or substituted ammonium salt, can be present in the composition. Exemplary of long chain alkyl or alkenyl are from 8 to 22 carbon atoms in length, specifically 10 to 20 carbon atoms in length, more specifically alkyl and most specifically normal, or normal with little branching. Small quantities of olefinic bond(s) may be present in the predominantly alkyl sections, particularly if the source of the "alkyl" group is obtained from a natural product such as tallow, coconut oil and the like.

Examples of anionic surfactants other than soap include but are not limited to alkyl sulfates, anionic acyl sarcosinates, methyl acyl taurates, N-acyl glutamates, acyl isethionates, alkyl sulfosuccinates, alkyl phosphate esters, ethoxylated alkyl phosphate esters, trideceth sulfates, protein condensates, and mixtures of ethoxylated alkyl sulfates.

Alkyl chains for these surfactants are $C_8$-$C_{22}$, preferably $C_{10}$-$C_{18}$, more preferably $C_{12}$-$C_{14}$. Anionic nonsoap surfactants can be exemplified by the alkali metal salts of organic sulfate having in their molecular structure an alkyl radical containing from 8 to 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$-$C_{18}$ carbon atoms), sodium coconut oil fatty acid monoglyceride sulfates and sulfonates; sodium or potassium salts of sulfuric acid esters of the reaction product of 1 mole of a higher fatty alcohol e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art.

Zwitterionic surfactants can be exemplified by those which can be broadly described as derivatives of aliphatic quaternary ammonium, phosphonium, and sulfonium compounds, in which the aliphatic radicals can be straight chain or branched and wherein one of the aliphatic substituents contains from 8 to 18 carbon atoms and one contains an anionic water-solubilizing group, e.g., carboxy, sulfonate, sulfate, phosphate, or phosphonate. A general formula for these compounds is:

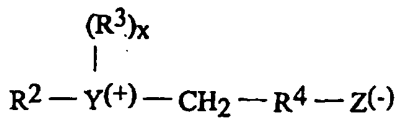

wherein $R^2$ contains an alkyl, alkenyl, or hydroxy alkyl radical of from 8 to 18 carbon atoms, from 0 to 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to 3 carbon atoms; X is 1 when Y is a sulfur atom and 2 when Y is a nitrogen or phosphorus atom, $R^4$ is an alkylene or hydroxyalkylene of from 0 to 4 carbon atoms and Z is a radical selected from the group consisting of carboxylate, sulfonate, sulfate, phosphonate, and phosphate groups.

Examples include: 4-[N,N-di(2-hydroxyethyl)-N-octadecylammonio]-butane-1-carboxylate; 5-[S-3-hydroxypropyl-S-hexadecylsulfonio]-3 hydroxypentane-1-sulfate; 3-[P,P-P-diethyl-P 3,6,9 trioxatetradecyl- phosphonio]-2-hydroxypropane-1-phosphate; 3-[N,N-dipropyl-N-3 dodecoxy-2-hydroxypropylammonio]-propane-1-phosphonate; 3-(N, N-di- methyl-N-hexadecylatnmonio)propane-1-sulfonate; 3-(N,N-dimethyl-N-hexadecylammonio)-2-hydroxypropane-1-sulfonate; 4-(N,N-di(2-hydroxyethyl)-N-(2 hydroxydodecyl)ammonio]-butane-1-carboxylate; 3-[S-ethyl-S-(3-dodecoxy-2-hydroxypropyl)sulfonio]-propane-1-phosphate; 3-(P,P-dimethyl-P-dodecylphosphonio)-propane-1-phosphonate; and 5-[N,N-di(3-hydroxypropyl)-N-hexadecylammonio]-2-hydroxy-pentane-1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from 8 to 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines, such as the one prepared by reacting dodecylamine with sodium isethionate according to the teaching of U.S. Patent No. 2,658,072, N-higher alkyl aspartic acids; such as those produced according to the teaching of U.S. Patent No. 2,438,091, in U.S. Patent No. 2,528,378. Other amphoterics such as betaines are also useful in the present composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl) carboxymethyl betaine, oleyl dimethyl gamma-carboxypropyl betaine, lauryl bis-(2-hydro-xypropyl) alpha-carboxyethyl betaine, etc. The sulfobetaines may be represented by coco dimethyl sulfopropyl betaine, stearyl dimethyl sulfopropyl betaine, amido

betaines, and amidosulfobetaines.

Many cationic surfactants are known to the art. By way of example, the following may be mentioned:

- stearyldimenthylbenzyl ammonium chloride;
- dodecyltrimethylammonium chloride;
- nonylbenzylethyldimethyl ammonium nitrate;
- tetradecylpyridinium bromide;
- laurylpyridinium chloride;
- cetylpyridinium chloride
- laurylpyridinium chloride;
- laurylisoquinolium bromide;
- ditallow(Hydrogenated)dimethyl ammonium chloride;
- dilauryldimethyl ammonium chloride; and
- stearalkonium chloride.

Additional cationic surfactants are disclosed in U.S. Patent No. 4,303,543 see column 4, lines 58 and column 5, lines 1-42. Also see CTFA Cosmetic Ingredient Dictionary, 4th Edition 1991, pages 509-514 for various long chain alkyl cationic surfactants.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration with ethylene oxide, e.g., a coconut alcohol ethylene oxide condensate having from 10 to 30 moles of ethylene oxide per mole of coconut alcohol, the coconut alcohol fraction having from 10 to 14 carbon atoms. Other ethylene oxide condensation products are ethoxylated fatty acid esters of polyhydric alcohols (e.g., Tween 20-polyoxyethylene (20) sorbitan monolaurate).

4. Long chain tertiary amine oxides corresponding to the following general formula:

$$R_1R_2R_3N \rightarrow 0$$

wherein $R_1$ contains an alkyl, alkenyl or monohydroxy alkyl radical of from 8 to 18 carbon atoms, from 0 to 10 ethylene oxide moieties, and from 0 to 1 glyceryl moiety, and, $R_2$ and $R_3$ contain from 1 to 3 carbon atoms and from 0 to 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyl-di(2-hydroxyethyl) amine oxide, dimethyloctylamine oxide, dimethyldecylamine oxide, dimethyltetradecylamine oxide, 3,6,9 trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

RR'R"P→0

wherein R contains an alkyl, alkenyl or monohydroxyalkyl radical ranging from 8 to 20 carbon atoms in chain length, from 0 to 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecylmethylethylphosphine oxide, 3,6,9-trioxaoctadecyldimethylphosphine oxide, cetyldimethylphosphine oxide, 3-dodecoxy-2-hydroxypropyldi(2-hydroxyethyl) phosphine oxide stearyldimethylphosphine oxide, cetylethyl propylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleyldimethylphosphine oxide, 2-hydroxydodecyldimethylphosphine oxide.

6. Long chain dialkyl sulfoxides containing one short chain alkyl or hydroxy alkyl radical of 1 to 3 carbon atoms (usually methyl) and one long hydrophobic chain which contain alkyl, alkenyl, hydroxy alkyl, or keto alkyl radicals containing from 8 to 20 carbon atoms, from 0 to 10 ethylene oxide moieties and from 0 to 1 glyceryl moiety. Examples include: octadecyl methyl sulfoxide, 2-ketotridecyl methyl sulfoxide, 3,6,9-trioxaoctadecyl 2-hydroxyethyl sulfoxide, dodecyl methyl sulfoxide, oleyl 3-hydroxypropyl sulfoxide, tetradecyl methyl sulfoxide, 3 methoxytridecylmethyl sulfoxide, 3-hydroxytridecyl methyl sulfoxide, 3-hydroxy-4-dodecoxybutyl methyl sulfoxide.

7. Alkylated polyglycosides wherein the alkyl group is from 8 to 20 carbon atoms, preferably 10 to 18 carbon atoms and the degree of polymerization of the glycoside is from 1 to 3, preferably 1.3 to 2.0.

Component b can be a typical hydrocarbonaceous material such as a wax, petrolatum, mineral oil, beeswax, a "permethyl" made up of longer chain branched hydrocarbons available from Permethyl Corporation. Permethyls are of the general formula

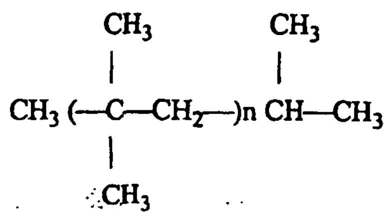

where n can vary from 4 to over 200. Products were n = 4, 16, 38, 214, respectively, are marketed as Permethyl 102A, 104A, 106A and 1082A.

Additional hydrocarbonaceous materials include lanolins, lanoleic materials, coco butter, shea butter and long chain alkyl esters and ethers of the lanolins.

The petrolatum useful in the present invention can be any grade of white or yellow petrolatum recognized in the art as suitable for human application. Preferred petrolatum are those with a melting point in a range of from 35°C to 70°C more preferably 50 to 60°C. The petrolatum of the composition can include hydrocarbon mixtures formulated with mineral oil and/or in combination with paraffin waxes of various melting points; all in small quantities compared to the petrolatum. A petrolatum without additional materials is preferred. Examples of waxes, particularly useful in solid compositions are microcrystalline waxes, generally those waxes which are known as paraffin wax, beeswax, natural waxes derived from vegetables, shea wax and the like.

Cationic polymers includes but are not limited to the following groups:

(i) cationic polysaccharides;
(ii) cationic copolymers of saccharides and synthetic cationic monomers, and
(iii) synthetic polymers selected from the group consisting of:

a. cationic polyalkylene imines
b. cationic ethoxy polyalkylene imines
c. cationic poly[N-[3-(dimethylammonio)propyl] N'[3-(ethyleneoxyethylene dimethylammonio)propyl]urea dichloride]

d. in general a polymer having a quaternary ammonium or substituted ammonium ion.

The cationic polysaccharide class encompasses those polymers based on 5 or 6 carbon sugars and derivatives which have been made cationic by engrafting of cationic moieties onto the polysaccharide backbone. They may be composed of one type of sugar or of more than one type, i.e. copolymers of the above derivatives and cationic materials. The monomers may be in straight chain or branched chain geometric arrangements. Cationic polysaccharide polymers include the following: cationic celluloses and hydroxyethylcelluloses; cationic starches and hydroxyalkyl starches; cationic polymers based on arabinose monomers such as those which could be derived from arabinose vegetable gums; cationic polymers derived from xylose polymers found in materials such as wood, straw, cottonseed hulls, and corn cobs; cationic polymers derived from fucose polymers found as a component of cell walls in seaweed; cationic polymers derived from fructose polymers such as Inulin found in certain plants; cationic polymers based on acid containing sugars such as galacturonic acid and glucuronic acid; cationic polymers based on amine sugars such as galactosamine and glucosamine; cationic polymers based on 5 and 6 membered ring polyalcohols; cationic polymers based on galactose monomers which occur in plant gums and mucilages; cationic polymers based on mannose monomers such as those found in plants, yeasts, and red algae; cationic polymers based on galactommannan copolymer known as guar gum obtained from the endosperm of the guar bean.

Specific examples of members of the cationic polysaccharide class include the cationic hydroxyethyl cellulose JR 400 made by Union Carbide Corporation; the cationic starches Stalok® 100, 200, 300, and 400 made by Staley, Inc.; the cationic galactomannans based on guar gum of the Galactasol 800 series by Henkel, Inc. and the Jaguar Series by Celanese Corporation.

The cationic copolymers of saccharides and synthetic cationic monomers useful in the present invention encompass those containing the following saccharides: glucose, galactose, mannose, arabinose, xylose, fucose, fructose, glucosamine, galactosamine, glucuronic acid, galacturonic acid, and 5 or 6 membered ring polyalcohols. Also included are hydroxymethyl, hydroxyethyl and hydroxypropyl derivatives of the above sugars. When saccharides are bonded to each other in the copolymers, they may be bonded via any of several arrangements, such as 1,4-$\alpha$; 1,4-$\beta$; 1,3$\alpha$; 1,3; 1,3$\beta$; and 1,6 linkages. The synthetic cationic monomers for use in these copolymers can include dimethyldiallylammonium chloride, dimethylaminoethylmethyacrylate, diethyldiallylammonium chloride, and N,N-diallyl,N-N-dialkyl ammonium halides. A preferred cationic polymer is Polyquaternium 7 prepared with dimethyldialkylammonium chloride and acrylamide monomers.

Still further examples of cationic polymers include the polymerized materials such as certain quaternary ammonium salts, copolymers of various materials such as hydroxyethyl cellulose and dialkyldimethyl ammonium chloride, acrylamide and beta methacryloxyethyl trimethyl ammonium methosulfate, the quaternary ammonium salt of methyl and stearyl dimethylaminoethyl methacrylate quaternized with dimethyl sulfate, quaternary ammonium polymer formed by the reaction of diethyl sulfate, a copolymer of vinylpyrrolidone and dimethyl aminoethylmethacrylate, quaternized guars and guar gums and the like. Exemplary of cationic polymers which can be used to make the complexes of this invention include, as disclosed in the CTFA International Cosmetic Ingredient Dictionary (Fourth Edition, 1991, pages 461 -464); Polyquaternium -1, -2, -4 (a copolymer of hydroxyethylcellulose and diallyidimethyl ammonium chloride), -5 (the copolymer of acrylamide and beta methacrylyloxyethyl trimethyl ammonium methosulfate), -6 (a polymer of dimethyl diallyl ammonium chloride), -7 (the polymeric quaternary ammonium salt of acrylamide and dimethyl diallyl ammonium chloride monomers, -8 (the polymeric 5 quaternary ammonium salt of methyl and stearyl dimethylaminoethyl methacrylate quaternized with dimethyl sulfate), -9 (the polymeric quaternary ammonium salt of polydimethylaminoethyl methacrylate quaternized with methyl bromide), -10 (a polymeric quaternary ammonium salt of hydroxyethyl cellulose reacted with a trimethyl ammonium substituted epoxide), - 11 (a quaternary ammonium polymer formed by the reaction of diethyl sulfate and a copolymer of vinyl pyrrolidone and dimethyl aminoethylmethacrylate), -12 (a polymeric quaternary ammonium salt prepared by the reaction of ethyl methacrylate/abietyl methacrylate/diethylaminoethyl methacrylate copolymer with dimethyl sulfate), -13 (a polymeric quaternary ammonium salt prepared by the reaction of ethyl methacrylate/oleyl methacrylate/diethylaminoethyl methacrylate copolymer with dimethyl sulfate), -14, -15 (the copolymer of acrylamide and betamethacrylyloxyethyl trimethyl ammonium chloride), -16 (a polymeric quaternary ammonium salt formed from methylvinylimidazolium chloride and vinylpyrrolidone), -17, -18, -19 (polymeric quaternary ammonium salt prepared by the reaction of polyvinyl alcohol with 2,3 epoxy-propylamine), -20 (the polymeric quaternary ammonium salt prepared by the reaction of polyvinyl octadecyl ether with 2,3-epoxypropylamine), -22, -24 (a polymeric quaternary ammonium salt of hydroxyethyl cellulose reacted with a lauryl dimethyl ammonium substituted epoxide), -27 (the block copolymer formed by the reaction of Polyquatemium-2 (q.v.) with Polyquaternium-17 (q.v.)), -28, -29 (is Chitosan (q.v.) that has been reacted with propylene oxide and quaternized with epichlorohydrin), and -30.

An additional component which is present, is a silicone. Silicone as used herein is preferably a silicone fluid, as opposed to a silicone gum. A silicone fluid is defined herein as silicone with viscosities ranging from 5 * $10^{-6}$ to 0.6 $m^2$/sec (5 to 600,000 centistokes), more preferably from 350 * $10^{-6}$ to 0.1 $m^2$/sec (350 to 100,000 centistoke) at 25°C. Polyalkyl siloxanes such as polydimethyl siloxane generally known as "dimethicone", are preferred for use as the

silicone.

The silicone materials useful in the present invention are generally non-volatile and may be either a polyalkyl siloxane, a polyaryl siloxane, a polyalkylaryl, a functionalized siloxanelated, a polysiloxane such as a polysiloxan with amino functional substitution, an alkoxylated silicone, such as ethoxylated or propoxylated, and a polyether siloxane copolymer. The silicones useful in the present invention may be endcapped with any number of moieties, including, for example, methyl, hydroxyl, ethylene oxide, propylene oxide, amino, trialkyl silane (preferably methyl), carboxyl, and the like. Mixtures of these materials may also be used and are preferred in certain implementations. Additionally, volatile silicones may be used as part of the silicone mixture so long as the final mixture is at least essentially non-volatile.

The polyalkyl silicones that may be used herein include, for example, polydimethyl siloxanes with viscosities ranging from $5*10^{-6}$ to 0.6 $m^2$/sec (5 to 600,000 centistokes) at 25°C. These siloxanes are available, for example, from General Electric Company as the Viscasil series and from Dow Coming as the Dow Coming 200 series. The viscosity can be measured by means of a glass capillary viscosmeter as set forth in Dow Coming Corporate Test Method CTM0004, Jul. 20, 1970. Preferably the viscosity ranges from $50* 10^{-6}$ to 0.15 $m^2$/sec (50 centistokes to 150,000 centistokes) and most preferably from $350*10^{-6}$ to 0.1 $m^2$/sec (350 centistokes to 100,000 centistokes).

The polyalkylaryl silicones that may be used include, for example, polymethylphenylsiloxanes having viscosities of from 15-65 $*10^{-6}$ $m^2$/sec (15 to 65 centistokes) at 25°C. These siloxanes are available, for example, from the General Electric Company as SF 1075 methyl phenyl fluid or from Dow Coming as 556 Cosmetic Grade Fluid. Additionally, poly(dimethyl siloxane) (diphenyl siloxane) copolymers having a viscosity in the range of from $10 * 10^{-6}$ to 0.1 $m^2$/sec (10 to 100,000 centistokes) at 25°C are useful. The polyether siloxane copolymer that may be used is, for example, a polypropylene oxide modified dimethylpolysiloxane (e.g., Dow Coming DC-1248, although ethylene oxide or mixtures of ethylene oxide and propylene oxide may also be used.

References disclosing suitable silicones include U.S. Patent No. 2,826,551, U.S. Patent No. 3,964,500, U.S. Patent No. 4,364,837, and British Patent No. 849,433. Also reference is made to Silicon Compounds, distributed by Petrarch Systems, Inc., 1984. This reference provides a good listing of suitable silicone material.

Other components can also be present in the composition. These components includes preservative(s), colorant(s), UV stabilizers, fragrance(s), antibacterial agent(s), and the like. Antibacterial agent(s) include chlorhexidine, triclosan, triclocarban and the like at their typically used concentration, i.e. 0.1 to 1.5 wt. %, preferably 0.15 to 1.2 wt. % of the composition. When present in the compositions of the invention, there is a dual effect. Not only is there an inhibition of germ attachment following the skin cleansing but there is also the antibacterial effect of the antibacterial agents upon bacteria present on the skin during skin cleansing.

The preferred surfactant is an anionic surfactant such as soap, alkylisethionate such as sodium cocoylisethionate, a sulfonate, and a sulfate (optionally ethoxylated). Mixtures of surfactants can be employed. There should be sufficient surfactant present to bring about a cleansing effect. The surfactant preferably anionic or mixtures thereof involving one or more from the other families of illustrated surfactants (amphoteric, nonionic and the like) with or without an additional anionic surfactant, can be present in the composition in various quantities. For example broad minimums of the surfactant can be present at 1, 2, 3, 4, 5, 10, 15 or 20 wt.% of the compositions, particularly where the aqueous composition is a liquid. With respect to liquid, aqueous, compositions, the anionic surfactant is from 2 to 25 wt.% of the composition, specifically 5 to 20 wt.%. Other surfactants may be present such as an amphoteric, particularly a betaine, and a nonionic, particularly an alkylated polyglycoside. Their quantities are from 1 to 20 wt.% of the composition. Generally the total surfactant in a liquid composition is at least 3 or 4 wt.%, preferably at least 5 wt.% and is generally no more than 30 wt.%, preferably no more than 25 wt.% but can be as low as no more than 10, 15 or 20 wt. %. For a solid composition, the total surfactant can be from 60 to 90 wt.%, preferably 70 to 85 wt.%, of the composition. Soap can be present at 15 to 100 wt.% of the total surfactant. "Soap-bars" generally have from 65 to 90 wt.% soap therein with less than 10 wt.%, preferably less than 5 wt.% of other surfactant therein. Most preferably, there is zero or zero to 2 wt.% of other surfactant therein. Bars having a smaller quantity of soap within the disclosed range of soap usually have a mild synthetic surfactant therein such as sodium cocoyl isethionate at moderate to high levels.

The quantity of hydrocarbonaceous component should be present in at least 0.1, preferably from 0.5 wt.% of the composition. Although the maximum can be up to 7 or 8wt.% of the composition, it is preferred to have a maximum of 5 wt.%, preferably 4.5 wt.% of the composition.

If a silicone is present in the composition, the minimum quantity is 0.01 wt.% of the composition, preferably at least 0.1 wt.%. The maximum can vary but generally is not above 7 or 8 wt.%, preferably 5 wt.%, more preferably 4.5 wt.% of the composition.

When using a cationic polymer in the composition, the quantity of polymer is from 0.01 to 3.0wt.% of the composition preferably 0.02 wt.% as a minimum and more preferably 0.03 wt.% as a minimum. The maximum is generally no more than 0.9 wt.%, or 0.75wt.%, although lower maximums such as 0.6wt.% can be employed.

The physical nature of the composition is not critical and can be a solid, liquid or gel.

The inhibition of germ attachment to the skin is quantitatively assessed by utilizing various bacteria in the protocol below. The enumerated test bacteria are employed. The number reflects the ATCC catalogue number:

- Staph. aureus 6538
- Staph. epidermidis 12228
- C. minutissimum, 23347
- E. coli 11229
- Serratia marcescens 14756
- Salmonella choleraesuis 10708

The test bacteria are radiolabeled in the following manner:

The test bacteria are grown in log phase in 30 ml of trypticase soy broth (TSB). Next day, bacteria are centrifuged at 3000 rpm for 20 min at 4°C. The bacterial pellet is resuspended in 20 ml of sterile saline and OD adjusted to 0.1 at 620 nm. Approximately, $10^5$ bacteria (0.1 ml) are inoculated in 5 ml of growth medium (2 ml of Methionine assay medium+ 3 ml of TSB) for radio-labeling. Fifty microliter of $^{14}C$ methionine (=5 uCi) are added into the tube and incubated overnight at 37°C in a shaker incubator. Next day, the bacteria are pelleted by centrifuging at 3 K rpm for 15 min at 4°C. This step is repeated for a total of three times to remove free $^{14}C$. Each time 25 µl of supernatant and 25 µl of resuspended pellets are collected in scintillation vials for activity measurement. Usually after 3 washes, very little free activity remain in the supernatant.

The following soap bars are prepared having approximately 10 wt.% water and 1% of fragrance. The control soap bar of 1% fragrance, 10 wt.% water and the remainder essentially soap is employed. The quantity of soap in the test bars below is reduced by the quantity of petrolatum, silicone and polyquaternium employed.

Petrolatum containing soap bar - 3.5 wt.% petrolatum from Penreco as Snow White petrolatum - mp of 50-60°C. Petrolatum plus silicone plus polyquat containing soap bar 3.5 wt.% petrolatum from Penreco as Snow White petrolatum - mp of 50-60°C. Dimethicone of 1 wt.% from GE has a viscosity of (60,000 centistokes) 0.06 $m^2$/sec. Polyquat-6 as Merquat 100 (active ingredient 40%) 0.6 wt.% of composition.

**Product preparation:**

5% solutions of the product are made in deionized water (weigh 5 g and dissolve in 100 ml of deionized water by gentle heating).

**Product application:**

Soak cotton ball with the product solution and rub on a pig skin piece approximately 2.5 cm obtained from the belly region for 15 sec and lather for 45 sec and finally rinse for 15 sec in running warm (30°C) tap water.

**Bacterial attachment:**

Wait till no moisture left on the skin. Apply 25 µl of labeled bacterial suspension all over the skin using a positive displacement pipette. Elute at 2 min after deposition or after 30 min of deposition) with 0.5 ml of letheen broth three times and collect in a scintillation vial.

**Control for inoculum:**

Transfer 25 µl of labeled bacteria into vials in triplicate for input control.

The results below show the quantity of bacteria left on the skin sample(s) following removal of the bacterial suspension after a contact time of 1-2 minutes or thirty (30) minutes.

TABLE I

| Bacteria | Product | Bacteria Remaining After Contact Time of 1-2 minutes | | Attached to Skin (% Bacteria Remaining) 30 minutes | | |
|---|---|---|---|---|---|---|
| *S. aureus* | Soap | 39,420 | (16.9) | 84,941 | (36.2) | comp. |
| 235,000 | Soap + 3.5% Petrolatum | 27,716 | (11.9) | 57,753 | (24.7) | comp. |

TABLE I (continued)

| Bacteria | Product | Bacteria Remaining After Contact Time of 1-2 minutes | | Attached to Sk minutes | | |
|---|---|---|---|---|---|---|
| | Soap + Pet. + Sil. + Polyquat | 18,098 | (7.8) | 51,495 | (22.0) | |
| | | | | | | |
| *S. epiderm* | Soap | 24,822 | (23.1) | 30,940 | (28.8) | comp. |
| 107,506 | Soap + 3.5% Petrolatum | 18,872 | (17.6) | 27,006 | (25.1) | comp. |
| | Soap + Pet. + Sil. + Polyquat | 16,842 | (15.7) | 19,908 | (18.5) | |
| | | | | | | |
| *C. minutis* | Soap | 61,950 | (25.7) | 104,895 | (43.6) | comp. |
| 240,000 | Soap + 3.5% Petrolatum | 40,110 | (16.6) | 59,265 | (24.6) | comp. |
| | Soap + Pet. + Sil. + Polyquat | 21,270 | (8.7) | 47,910 | (19.8) | |
| | | | | | | |
| *E. coli* | Soap | 50,508 | (19.0) | 66,544 | (25.0) | comp. |
| 266,000 | Soap + 3.5% Petrolatum | 14,972 | (5.6) | 40,956 | (15.4) | comp. |
| | Soap + Pet. + Sil. + Polyquat | 3,360 | (1.3) | 13,772 | (5.2) | |
| | | | | | | |
| *S. marcescen* | Soap | 31,892 | (10.8) | 61,368 | (20.8) | comp. |
| 295,000 | Soap + 3.5% Petrolatum | 5,948 | (2.0) | 21,034 | (7.1) | comp. |
| | Soap + Pet. + Sil. + Polyquat | 5,260 | (1.8) | 13,888 | (4.7) | |
| | | | | | | |
| *S. choleraesu* | Soap | 12,574 | (9.2) | 13,912 | (10.2) | comp. |
| 236,000 | Soap + 3.5% Petrolatum | 6,574 | (4.8) | 17,893 | (13.2) | comp. |
| | Soap + Pet. + Sil. + Polyquat | 5,512 | (4.1) | 10,132 | (7.4) | |

comp : comparative example

These data clearly show that the presence of petrolatum inhibits the attachment of various bacteria to the skin. The additional presence of a silicone and a cationic polymer further inhibits the attachment of bacteria to the skin.

As well as the rinse off compositions, the effect of inhibition of germ attachment also occurs with leave on compositions such as creams, lotions, and the like. These latter compositions are characterized by the fact that they

are intended to be left on the skin for an extended period of time as opposed to an ordinary cleansing composition which is rinsed off by water after a relatively short contact time with the skin. After rinsing these "leave on" compositions from the skin, inhibition of germ attachment to the skin occurs. Desirable is the inhibition of attachment of bacteria to the skin. There need not be a surfactant in cleansing amounts present in the "leave on". Below are exemplary compositions of the "leave on" compositions of the invention.

| LOTION | |
|---|---|
| **Ingredient** | **%** |
| Water | 81.41 |
| Petrolatum | 3.00 |
| Magnesium Aluminum Silicate | 0.08 |
| Glycerin | 2.60 |
| Glyceryl/PEG-100 Stearate | 1.60 |
| Sodium Cetearyl Sulphate | 0.32 |
| Cetearyl Alcohol | 0.60 |
| Mineral Oil-Light | 4.00 |
| Dimethicone | 0.80 |
| Tocopheryl Acetate | 0.50 |
| Isopropyl Palmitate | 2.60 |
| Carbomer 2984 | 0.30 |
| Deionized Water | 1.00 |
| 99% Triethanolamine | 0.30 |
| Phenoxyethanol | 0.15 |
| Methyldibromo Glutaronitrile | 0.10 |
| Fragrance | 0.30 |
| Polysorbate 60 | 0.16 |
| Vitamin A Palmitate | 0.08 |
| D Panthenol 50-P | 0.10 |
| Total | 100.00 |

| CREAM | |
|---|---|
| Ingredient | % |
| Water | 80.60 |
| Petrolatum | 2.25 |
| Magnesium Aluminum Silicate | 0.10 |
| Glycerin | 2.00 |
| Glyceryl Stearate/PEG-100 Stearate | 2.00 |
| Sodium Cetearyl Sulphate | 0.32 |
| Isohexadecane | 1.50 |
| Cetyl-Stearyl Alcohol 50-50 | 0.75 |
| Mineral Oil-Light | 5.00 |
| Dimethicone | 1.00 |
| Tocopheryl Acetate | 0.50 |
| Isopropyl Palmitate | 2.00 |
| Carbomer 2984 | 0.36 |
| 99% Triethanolamine | 0.36 |
| Fragrance | 0.30 |
| Phenoxyethanol | 0.15 |
| Methyldibromo Glutaronitrile | 0.10 |
| Polysorbate 60 | 0.20 |
| Vitamin A Palmitate | 0.01 |
| D Panthenol 50-P | 0.50 |
| Total | 100.00 |

The liquid compositions of the invention in either rinse off or leave on formulations are oil-in-water emulsions. They, as well as the solid compositions, perform their inhibition of attachment of germs without any topically active drugs or medication present in the compositions, particularly those drugs or medications associated with relieving or minimizing conditions in the target group such as atopic dermatitis, psoriasis, and immunodeficient conditions. Examples of such agents include amcinonide, diflorasone diacetate, and hydrocortisone for dermatitis; and anthralin, methoxsalen, and coal tar the like for psoriasis. Therefore, such topical agents and medications can be desirably left out completely from all the compositions of this invention or can be present in the composition in amounts which are not sufficient to perform their intended function with respect to the target group, particularly those having atopic dermatitis, psoriasis and immunodeficient conditions.

## Claims

**1.** Use of a composition comprising

(a) a skin cleansing effective amount of 3 to 30 wt.% when used in a liquid composition, or of 60 to 90 wt.% when used in a solid composition of a surfactant or mixture of surfactants;
(b) a hydrocarbonaceous component selected from wax, petrolatum, mineral oil, beeswax, permethyl, lanolin, lanoleic material, coco butter, shea butter and long chain alkyl ester and ether of lanolin in amounts of 0.1 to 8 wt.% of the composition effective to inhibit attachment of germs to the skin;
(c) a cationic polymer; and
(d) a silicone;

for the preparation of a skin cleansing material which inhibits the attachment of germs to the skin.

**2.** The use in accordance with claim 1, wherein said composition can be rinsed from the skin with water.

**3.** The use of the composition in accordance with claims 1 or 2 for the preparation of a skin cleansing material which inhibits the attachment of germs to the skin of people having a skin condition selected from the group consisting of atopic dermatitis, psoriasis, or immunodeficient condition.

**4.** The use of the composition in accordance with claim 1, 2 and 3 wherein (b) is present in from 0.5 to 7 wt.% of the composition.

**5.** The use of the composition in accordance with claim 1 wherein the cationic polymer is present in 0.01 - 3 wt.% of the composition.

**6.** The use of the composition in accordance with claim 1 wherein the silicone is present in 0.1 to 8 wt.% of the composition.

**Patentansprüche**

**1.** Verwendung einer Zusammensetzung, die

(a) eine hautreinigend wirkende Menge von 3 bis 30 Gew.-% eines Tensids oder einer Mischung von Tensiden, wenn es/sie in einer flüssigen Zusammensetzung verwendet wird/werden, oder von 60 bis 90 Gew.-% eines Tensids oder einer Mischung von Tensiden, wenn es/sie in einer festen Zusammensetzung verwendet wird/werden,
(b) eine kohlenwasserstoffhaltige Komponente ausgewählt aus Paraffin, Petrolatum, Mineralöl, Bienenwachs, Permethyl, Lanolin, Lanolinmaterial, Koskosnussbutter, Shea-Butter (Butyrospermum parkeii-Butter) sowie langkettige Alkylester und Ether von Lanolin in Mengen von 0,1 bis 8 Gew.-% der Zusammensetzung, die wirksam sind, um eine Haftung von Keimen auf der Haut zu hemmen,
(c) kationisches Polymer und
(d) Silikon,

umfasst, zur Herstellung eines hautreinigenden Materials, das die Haftung von Keimen an der Haut hemmt.

**2.** Verwendung nach Anspruch 1, bei der die Zusammensetzung mit Wasser von der Haut abgespült werden kann.

**3.** Verwendung der Zusammensetzung nach den Ansprüchen 1 oder 2 zur Herstellung eines hautreinigenden Materials, das die Haftung von Keimen an der Haut von Leuten mit einer Hauterkrankung ausgewählt aus der Gruppe bestehend aus atopischer Dermatitis, Psoriasis oder einem Immundefekt hemmt.

**4.** Verwendung der Zusammensetzung nach Anspruch 1, 2 und 3, bei der (b) in Form von 0,5 bis 7 % der Zusammensetzung vorhanden ist.

**5.** Verwendung der Zusammensetzung nach Anspruch 1, bei der das kationische Polymer in 0,01 bis 3 Gew.-% der Zusammensetzung vorhanden ist.

**6.** Verwendung der Zusammensetzung nach Anspruch 1, bei der das Silikon in 0,1 bis 8 Gew.-% der Zusammensetzung vorhanden ist.

**Revendications**

**1.** Utilisation d'une composition comprenant :

(a) une quantité efficace pour nettoyer la peau de 3 à 30 % en poids quand il est utilisé dans une composition liquide, ou de 60 à 90 % en poids quand il est utilisé dans une composition solide d'un tensioactif ou d'un mélange de tensioactifs ;
(b) un composant hydrocarboné choisi parmi une cire, un pétrolatum, une huile minérale, une cire d'abeille, un perméthyle, la lanoline, une substance lanoléique, le beurre de coco, le beurre de Karité, un ester à longue chaîne alkyle de lanoline et un éther à longue chaîne alkyle de lanoline en des quantités de 0,1 à 8 % en poids de la composition efficaces pour inhiber la fixation de germes sur la peau ;
(c) un polymère cationique ; et
(d) un silicone ;

pour la préparation d'une substance nettoyante pour la peau qui inhibe la fixation des germes sur la peau.

2. Utilisation selon la revendication 1, dans laquelle ladite décomposition peut être rincée de la peau avec de l'eau.

3. Utilisation de la composition selon la revendication 1 ou 2 pour la préparation d'une substance nettoyante pour la peau qui inhibe la fixation de germes sur la peau de personnes ayant une maladie de peau choisie dans le groupe constitué par la dermite atopique, le psoriasis, ou un état immunodéprimé.

4. Utilisation de la composition selon les revendications 1, 2 et 3 dans laquelle (b) est présent de 0,5 à 7 % en poids de la composition.

5. Utilisation de la composition selon la revendication 1 dans laquelle le polymère cationique est présent de 0,01 à 3 % en poids de la composition.

6. Utilisation de la composition selon la revendication 1 dans laquelle le silicone est présent de 0,1 à 8 % en poids de la composition.